**Europäisches Patentamt**

**European Patent Office**

**Office européen des brevets**

(11) Publication number: **0 166 623**
**A2**

# EUROPEAN PATENT APPLICATION

(21) Application number: 85304641.5

(51) Int. Cl.⁴: **G 01 N 33/543**

(22) Date of filing: 28.06.85

(30) Priority: 29.06.84 US 626003

(43) Date of publication of application: 02.01.86
Bulletin 86/1

(84) Designated Contracting States: **DE FR GB SE**

(71) Applicant: **ORTHO DIAGNOSTIC SYSTEMS INC., One Johnson & Johnson Plaza, New Brunswick New Jersey 08933 (US)**

(72) Inventor: **Thompson, Russell E., 22 Proctor Street, Marshield Massachusetts 02050 (US)**
Inventor: **Bander, Neil H., 1385 York Avenue Apt. 4G, New York New York 10021 (US)**

(74) Representative: **Jones, Alan John et al, CARPMAELS & RANSFORD 43 Bloomsbury Square, London, WC1A 2RA (GB)**

(54) Antibody lectin sandwich assay.

(57) Methods for detecting glycoproteins, glycopeptides, carbohydrates or glycolipids having sugar residues in an aqueous sample. The glycoprotein, etc., is initially selected by immunological reaction with an immobilized antibody and then detected by nonspecific reaction with a labeled lectin.

-1-

ANTIBODY-LECTIN SANDWICH ASSAY

Field of the Invention

This invention relates to diagnostic methods in general
and in particular, provides methods employing components
specific for materials to be assayed, and nonspecific
components for labeling.

Background of the Invention

The detection of specified antigens (defined as a
substance whose introduction into an animal stimulates the
production of antibodies capable of immunologically
reacting specifically therewith), haptens (a substance
requiring additional accessory materials before its
introduction into an animal stimulates the production of
antibodies specific therefor), and the like substances
(hereinafter collectively referred to as "ligands") in
body fluids such as blood, sputum, urine, and the like has
in recent years become of utmost importance in both the
research and clinical environments. The detection of such
ligands can often be related to various disease states and
consequently, is of extreme usefulness in diagnosis as
well as gaining basic understanding concerning the genesis
of disease as well as monitoring the effectiveness of
therapies therefor.

Accordingly, improved methods for detecting ligands in
aqueous samples are constantly sought. In particular,
such methods or assays are typically characterized by
their speed and facility of employment, as well as by
their sensitivity. Preferred assays are those requiring
less effort to perform in shorter time periods and
characterized by greater sensitivity. These aspects are,

L2

of course, modulated by costs, generally in turn regulated by the difficulty associated in manufacturing the assay.

It is an object of the present invention to provide generic and specific assays having sensitivity suitable for use in the clinical and research laboratory which are simpler in nature thereby resulting in lower costs and greater ease in manufacture.

Immunoassays in general are based upon the immunological reaction between proteins such as antibodies, antibody fragments, or even artificially generated peptides simulating antibody binding sites (hereinafter collectively referred to as "ligand binding partners") and the substance for which they are specific, i.e. the ligands. Immunological reactions are characterized by their high specificity and accordingly, numerous schemes have been developed in order to take advantage of this characteristic. Typically, such schemes require either purified antigen or ligand to compete with the ligand being measured, and a labeled and immobilized ligand binding partner; or multiple immobilized and labeled ligand binding partners reactive with themselves and the ligand.

Those techniques which require purified ligand to compete with the sample ligand for the binding site on the ligand binding partner disadvantageously entailed difficult and expensive manufacturing processes in order to produce the purified ligand in a form that is sufficiently intact to permit linkage with its specific binding partner. The production difficulties are exacerbated with small and generally uncharacterized ligands.

It is an object of the present invention to provide assay methods which do not rely upon competition between ligands or ligand binding partners for their respective "mates".

RL-12

0166623

In the traditional heterogeneous forward assays, an immobilized ligand or ligand binding partner (hereinafter, ligand or ligand binding partner are to be used interchangeably as either one of these components may be assayed for in a sample, it being understood that if a ligand is being assayed for, it will be combining with a ligand binding partner and vice versa) is immobilized on a solid phase. Such a solid phase may take a variety of forms and includes for instance microparticles, plastic surfaces such as those forming microtiter wells, paddles, microparticles, macroparticles and the like.

It is an object of the present invention to provide assays which can be advantageously used with such a variety of solid phases.

The sample is then contacted with the immobilized ligand binding partner and ligands, if present in the sample, specifically react therewith and become themselves immobilized. Unreacted components are typically removed and subsequently, labeled ligand binding partners, specific for a different antigenic determinant (i.e. binding site) on the ligand or alernatively, specific for a binding site on the immobilized ligand binding partner (and thus blocked from reacting by the presence of ligand), are added.

The second ligand binding partner typically has a detectable entity associated directly or indirectly therewith. Such detectable entities include fluorescent molecules, chemiluminescent molecules, enzymes, isotopes, microparticles and the like. These second labeled binding partners are permitted to react with the immobilized components and subsequently, unreacted components are removed. The detectable entity associated with the solid phase or aqueous phase is detected and related to the presence of ligand in the original sample. The average laboratory technician is quite conversant with such techniques, it being another object of the present invention to capitalize on this level of familiarity and acceptability.

The ligand binding partners, if they are antibodies or fragments thereof, may be of either polyclonal or monoclonal origin depending upon the availability of such antibodies, their specificity, avidity characteristics and the like.

Such conventional forward assays, however, require a multi-determinant ligand, and thus the selection, purification and production of multiple antibodies of differing specificities. Since one of the antibodies must be labeled, additional components are required with indirect labeling or, in the case of direct labeling, chemical manipulations which often result in poor immunological characteristics of the second binding partner. As a result, optimal sensitivity is often greatly impaired by weakened reactivities and by nonspecific reactions which are often very difficult to avoid. Certainly the costs and complexities associated with obtaining carefully matched antibody pairs is a significant deterrent to their development. Further, although it is usually possible to obtain at least one "acceptable" (based on specificity, avidity and the like characteristics) monoclonal antibody specific for a ligand, the level of difficulty of obtaining more than one acceptable antibody to different epitopes on the same ligand is dramatically greater and, of course, impossible with small ligands having only a single epitope.

It is yet another object of the present invention to provide forward, heterogeneous assay methods which do not rely upon immunological pairs.

It is still yet another object of the present invention to provide a labeling agent which does not rely upon an immunological reaction.

Several attempts have been made to employ lectins in an assay. For instance, U.S. Patent No. 4,298,689 to Doyle et al., describes a test for Neisseria gonorrhoeae utilizing a plant lectin having an affinity for N-acetylglucosamine. The sample suspected of containing the Neisseria gonorrhoeae microorganism is contacted directly with the plant lectin and the presence of reaction products formed on the cell surface of the microorganism is used as presumptive evidence of the organisms presence. This test relies upon a very specific type of plant lectin, one having a particular affinity for N-acetylglucosamine. In effect, the Doyle et al. method employs lectin as if it were a dye. There is no immunological selection process being carried on and accordingly, the Doyle et al. method provides very limited versatility.

It is an object of the present invention to provide more specific methods for the detection of a broader range of ligands, and to capitalize on the advantages provided by lectins.

U.S. Patent No. 4,389,392 to Adachi shares similar disadvantages with the Doyle et al. methods in that it requires the use of particular lectins which specifically combine with a terminal galactose β1 or β1-4-N-acetylglucosamine or -N-acetylgalactosamine in order to detect tumor associated glycolinkage containing substances. Adachi's methods however fail to utilize specificity advantages provided by immunological systems.

Thus, as a principle for maximizing utility and the versatility of reagents, it is an object of the present

12

invention to provide hybrid methods utilizing separately, both immunoglobulins and lectins thus combining advantages offered by each.

U.S. Patent Nos. 4,289,747 and 4,371,515 to Chu utilize a different approach to employing lectins. Chu conjugates a lectin with an immunoglobulin and then utilizes the lectins affinity for sugar to immobilize the conjugate such as when the sugar is insolubilized. Other variations of this approach are also described, however, they all disadvantageously entail lectin immunoglobulin conjugates or multiple immunoglobulins with complicated insolubilization schemes.

It is yet another object of the present invention to avoid the complexities such as those described by Chu in formulating an assay employing lectin.

It is still yet another object of the present invention to employ lectin which is not conjugated to an immunoglobulin and which is not employed to insolubilize immunologically reacted components.

It is a yet further object of the present invention to provide comparatively simpler assay methods employing lectin and only a single immunologically specific component.

It is a still yet further object of the present invention to provide assay methods which can rely upon nonimmunological but specifically reactive components such as cofactors, and receptors along with a lectin.

RL-12

0166623

-7-

## Summary of the Invention

In accordance with the principles and objects of the present invention, there are provided assay methods which employ an immobilized, immunologically active component for specifically selecting the material to be assayed from an aqueous sample. Unreacted components are preferably removed and a subsequent, nonspecific labeled agent added to react therewith via non-immunological mechanisms. Unreacted agent is preferably removed and the label associated with the agent in the solid phase or aqueous phase detected and correlated to the presence of the material or ligand to be determined in the original sample.

The labeled agent does not rely upon immunological processes for reaction, is simpler to produce, has greater storage life than presently used materials such as the second antibody of an immunological pair, and can be employed generally with particular classes of ligands thus reducing the number of specific reagents otherwise necessary to perform a variety of assays. As used herein, the term "nonspecific" is used to modify and describe the labeled agent, ideally a lectin since the lectin does not rely on immunological principles for selective reactivity. Thus, in comparison to an immunoglobulin, it is nonspecific since it will react with its respective sugar residue (or residues) whether or not it is part of the ligand to be determined. It should be appreciated, however, that different lectins are reactive with different sugar residues, albeit often demonstrating a higher level of binding when a plurality of sugar residues are available.

-8-

Brief Description of the Drawings

Further understanding of the principles and scope of the present invention may be had by reference to the drawings wherein:

FIGURE 1 shows $S_{23}/S_{27}$ Assay results;

FIGURE 2 compares the $S_{23}/S_{27}$ Assay against the $S_{27}$/Lectin Assay;

FIGURE 3 traces Antigen Levels in a transplant patient by both $S_{23}/S_{27}$ and $S_{27}$/Lectin Assays; and

FIGURE 4 compares the Assay results shown in FIGURE 3.

Detailed Description of the Invention and Best Mode

The immunization procedures for the production of polyclonal antibodies specific for particular antigens are well-known and need not be repeated here. Similarly, the procedures for obtaining monoclonal antibodies specific for particular antigens pursuant to the methods of Kohler and Milstein are also well-known. Such processes may be employed to obtain antibodies of desired specificity and avidity for immobilization upon a solid phase. The immobilization techniques will depend largely upon the nature of the solid phase selected but are also typically well-known and need not be detailed at length here.

Having immobilized an antibody, or antibody fragment, specific for the ligand of interest into a solid phase, the aqueous sample containing the ligands to be detected is then contacted with the immobilized ligand binding partner under conditions suitable for permitting an immunological reaction to occur therebetween. Such conditions are, of course, also known to those conversant in the art and generally include, for instance, room temperature, appropriate reaction times in view of concentrations, volumes, distances between components, physiological pHs, etc. After allowing contact for a time periods sufficient to permit immunological immobilization of any desired ligand present in the sample onto the surface, the aqueous sample and any unreacted components contained therein are preferably removed by siphoning, dumping, blotting, washing or any other mechanical expedient. As will be readily appreciated, superior technique in removal of unreacted components reduces nonspecific reactions and increases sensitivity.

Thereafter, the nonspecific, labeled agent is contacted with the solid phase, and any ligand associated therewith, and permitted to react. The nonspecific agent is preferably formulated so as to react only with ligand if present, and not with the immobilized ligand binding partner thereby advantageously avoiding nonspecific reactions and enhancing sensitivity.

In general accordance with conventional assays, the label may be selected from any of a variety of markers available for this purpose including fluorescent molecules, phosphorescent molecules, chemiluminescent molecules, radioisotopes, synthetic and natural polymeric particles of micro or macro dimension, light scattering particles and enzymes. With enzymes, it is to be understood that after performing the above steps, a substrate must be added upon which the enzyme can act to produce a detectable product. Alternatively, the label may be associated with a binding partner which reacts specifically with the nonspecific agent in the so-called indirect labeling format as exemplified by the procedure of Example 1. This route is, however, generally less preferred due to the increased number and complexity of reagents and process steps. Rather, and in accordance with the objects of the present invention, the nonspecific agent is itself directly labeled with a label preferably chosen to allow for facile detection employing inexpensive equipment such as set forth in Example 2 hereof.

In a preferred embodiment, the labeling system would be one relying upon fluorescence produced by fluorescent molecules such as fluorescein, rhodamine or the like. Alternatively, a simple enzyme system employing horseradish peroxidase enzyme linked to the nonspecific agent could be used in the manner of the ELISA type assay systems. Thereafter, o-phenylenediamine is supplied as a substrate and the appearance of product detected by optical density measurements at approximately 490 nm.

The instant invention specifically contemplates the detection of ligands such as glycoproteins, glycopeptides, carbohydrates, or glycolipids which contain at least one sugar residue recognized by a lectin. The solid phase ligand binding partner, preferably an antibody of monoclonal origin since its specificity can be carefully tailored, is selected in accordance with its specificity for some portion of the protein, carbohydrate, peptide or lipid backbone in the ligand. In contrast, the second agent or lectin of the present invention is "nonspecific" in the sense that it binds to every available, exposed sugar residue(s) which it normally specifically binds and which may be present within the sample. The preferred nonspecific agent is a lectin such as lentil lectin, peanut lectin, conconavalin A, or wheat germ lectin (or agglutinin) among others.

Due to the lack of "specificity" of the lectin, i.e., it discriminates only between sugar residues and not between ligands and non-ligands, a separation step prior to the addition of the labeled lectin is advantageously performed in order to reduce nonspecific reaction with other sugar residue containing materials present in the sample which have not reacted immunologically. Further nonspecific reactivity can be limited or eliminated entirely by employing suitable washes having suitable detergent and salt concentrations to assist in the removal of unwanted components. Further understanding in this regard will be had by reference to the Examples provided herein.

Although the preferred embodiment and Examples utilize the specificity provided by a monoclonal immunoglobulin, other specific binding moieties are contemplated including analyte receptors, cofactors and complement components.

Ueda et al. at Sloan-Kettering Cancer Center described the isolation and reactivity of seventeen monoclonal antibodies derived from fusions of spleen cells of mice immunized with established culture lines of renal cancers (Cell Surface Antigens of Human Renal Cancer Defined by Mouse Monoclonal Antibodies: Identification of Tissue-specific Kidney Glycoproteins, Proc. Natl. Acad. Sci. Vol. 78: 5122-5126, 1981). Two of the antibodies, $S_{23}$ and $S_{27}$, discovered by the Ueda et al. are the subject matter of a pending U.S. patent application, entitled "Disease Diagnosis by Detection of Shed Normal Tissue Antigens" to Thompson et al. Serial number 616 991. These antibodies have also been used herein in the Examples, but it is to be understood that the instant invention is in no way limited to the use of these particular antibodies. Although the referenced application completely describes the antibodies, background is also provided here for convenient reference. The $S_{23}$ and $S_{27}$ antibodies are the

result of hybridoma cell lines deposited in the American Type Culture Collection as ATCC No. HB8540 and ATCC No. HB8428, respectively. (It will be noted parenthetically that the Ueda et al. article cited earlier describes an $S_6$ antibody which has subsequently been replaced with an antibody, $S_{27}$, having substantially the same reactivity as the $S_6$ antibody but with preferable binding characteristics and is accordingly preferred herein.)

The $S_{23}$ and $S_{27}$ antibodies, and their equivalents, may also be characterized by their reactivity with a number of cell lines available from either the ATCC or Memorial Sloan-Kettering Cancer Center as set forth in Table 1.

In short, the $S_{23}$ antibody is an $IgG_1$ isotype formed from immunizing cell line SK-RC-7 (renal cancer) having observed frozen tissue section reactivity with the epithelial cells of proximal tubules in the human kidney. The $S_{27}$ antibody is similar to the $S_{23}$ but is also seen to react with some frozen tissue portions of the loop of Henle in the human kidney. Reactivities were determined with immunofluorescence staining techniques and direct microscopic observation. Both antibodies identify a 120,000 mw glycoprotein and demonstrate minimal cross-reactivities with other human tissue. The $S_{23}$ and $S_{27}$ antibodies are individually commercially available in unconjugated form from the assignee hereof under the trademarks ORTHO URO-4a and ORTHO URO-4.

### TABLE 1

| Cells[2] | Ab $S_{23}$ | | Ab $S_6$[1] | |
|---|---|---|---|---|
| | Titer x10$^{-3}$ | Abs. | Titer x10$^{-3}$ | Abs. |
| **Epithelial cancers:** | | | | |
| Renal | | | | |
| SK-RC-1 (AA) | 1 | + | 50 | + |
| SK-RC-2 (AB) | – | – | 50 | + |
| SK-RC-4 (AE) | 50 | + | 50 | + |
| SK-RC-6 (AG) | 10 | | 1000 | + |
| SK-RC-7 (AX) | 1 | + | 500 | + |
| SK-RC-8 (BE) | 1 | + | 50 | + |
| SK-RC-9 (BM) | – | + | 500 | + |
| SK-RC-11 (BZ) | 1 | + | 1000 | + |
| SK-RC-21 (EB) | – | – | 500 | + |
| SK-RC-28 (EU) | 500 | + | 5000 | + |
| SK-RC-29 (BW) | – | – | 50 | + |
| A-498 | – | + | 50 | + |
| CaKi-1 | – | – | 50 | + |
| Bladder | | | | |
| RT-4 | – | – | – | – |
| 5637 | – | – | – | – |
| T-24 | – | – | 5 | + |
| 253J | – | – | 5 | + |
| Breast | | | | |
| AlAb | – | – | – | – |
| BT-20 | – | – | – | – |
| MCF-7 | – | – | – | – |
| SK-BR-3 | – | – | – | – |
| Cervix | | | | |
| ME-180 | – | – | – | – |
| Colon | | | | |
| HT-29 | – | – | 5 | + |
| SW-1222 | – | – | – | – |
| Lung | | | | |
| SK-LC-LL | – | – | – | – |
| SK-LC-6 | – | | 50 | + |
| Ovary | | | | |
| SK-OV-3 | – | – | – | – |

| | C1 | C2 | C3 | C4 |
|---|---|---|---|---|
| Testicular | | | | |
| SK-GR-1 | − | − | − | − |
| Astrocytomas: | | | | |
| AJ, AS, BE | − | − | 5 | + |
| Melanomas: | | | | |
| SK-MEL-13,28,29,37,41 | − | − | − | − |
| SK-MEL-19 | − | − | − | |
| Neuroblastomas: | | | | |
| SK-NMC, SK-NSH | − | − | − | − |
| Lymphoblastoid cells: | | | | |
| EBV B cells | | | | |
| AX, BE, EU | | − | | |
| Burkitt's lymphomas | | | | |
| Raji, Daudi | | − | | − |
| T cells | | | | |
| MOLT-4, T-45 | | − | | − |
| Normal human cells: | | | | |
| Kidney epithelium | | | | |
| ID | 10 | + | 5 | + |
| EQ, HY | 1.5 | + | 5 | + |
| GM, FR | 3 | + | 5 | + |
| EI, IJ | 1.5 | + | 5 | + |
| EG, GR, IB | 0.5 | + | 5 | + |
| Fetal kidney | | | | |
| C-4, C-3 | − | − | >5 | + |
| C-6 | − | − | >5 | + |

[1] Subsequent to this publication, Ueda et al. discovered an alternative antibody, $S_{27}$, having substantially the same reactivity as $S_6$ but having preferable binding characteristics and accordingly $S_6$ has been replaced with $S_{27}$ both in their studies and herein.

[2] Generally available from either ATCC, Memorial Sloan-Kettering Cancer Center, or a number of other academic institutions.

RL-12

Example 1

I.  Immunization and Antibody Production

Generation of murine monoclonal antibodies have been described by Ueda et al., supra in detail.  Briefly, (BALB/c x C57BL/6)Fl mice were immunized with established renal cancer cell lines.  Spleen cells were harvested and fused with MOPC-21 NS/1 myeloma cells in the presence of polyethylene glycol.  Hybridoma cultures were cloned by limiting dilution until stable lines were obtained. Antibodies were characterized against a panel of cell lines and against tissue specimens as set forth in Table 1.  Ascites was produced by injecting the hybridoma lines subcutaneously into nu/nu mice of Swiss background.

II.  Monoclonal Antibody Purification

$S_{27}$ was purified from ascitic protein using a Protein A affinity column pursuant to the methods of Ey et al. (Immunochemistry 15:429-36, 1978) and Seppala et al. (Scand. J. Immunol. 14:335-42, 1981).  Antibodies were eluted from the column with a 0.01M citrate buffer, pH 4.5.  Alternatively, antibodies can be purified on a DEAE Affi-Gel blue column using the procedure of Bruck et al. (J. of Immunol. Methods 53:313-19, 1982).  Antibody activity was monitored against the renal cell line, SR-RC-7, by a two-step immunofluorescent procedure, using a fluorescein labeled goat anti-mouse IgG.  Evaluation of fluorescent intensity was made using the ORTHO Spectrum III™ flow cytometer.

One skilled in the art will readily appreciate that various alternatives and substitutions may be made regarding the type of ligand to be detected, the selection of the nonspecific binding component and in particular,

RL-12

-16-

different types of lectins, without departing from either the spirit or scope of the present invention.

### III. Lectin Detection System

Wheat Germ Agglutinin (WGA) isolated from Triticum vulgaris and modified with biotin (bWGA) (Vector Laboratories Inc.) was used in the detection of specifically bound antigen. Avidin conjugated to horseradish peroxidase (Avid-HRP) (Vector Laboratories Inc.) was used for the detection of bound lectin. Optimal signal to noise ratios were determined by titering out the bWGA and Avid-HRP conjugate versus nonspecific mouse IgG, of the gamma 1 isotype, and $S_{27}$ coated microtiter plates in the assay described below. Using lot number 20522 of bWGA and lot number 30422 of Avid-HRP, concentrations of 25 ng/ml and 625 ng/ml respectively were found to be acceptable.

### IV. Solid Phase Configuration

Purified $S_{27}$ was passively adsorbed onto the surfaces of Immunlon 2 microtiter plates (Dynatech Corporation). 50 ul of $S_{27}$ at a concentration of 10 ug/ml in 0.01 M Na2CO3/NaHCO3 buffer was allowed to adsorb overnight at 4°C. The plates were then washed with a phosphate buffered saline solution containing 0.3% Tween 20 and 0.45 M sodium chloride (PBST). Any remaining binding sites were blocked with 1% bovine serum albumin in phosphate buffered saline (PBS). The plates are washed again with PST prior to use.

Purified nonspecific mouse IgG, gamma 1 isotype, was used as a control for nonspecific binding. Coating and washing procedures were identical to those previously described above for $S_{27}$.

CRL-12

## V.   Standard Urine Preparation

Standards were prepared from a pool of patient urines with optical density values exceeding 2.0 in the assay described below.  The standard was aliquoted and stored at -70°C until used in the assay.  Assay standards with different levels of antigen were prepared from the standard urine by dilution in 0.1% bovine serum albumin in PBS.

## VI.   Sample Urine Handling

Urines were collecte from both normal individuals and individuals suffering from kidney diseases.  Sodium azide was added to a final concentration of 0.02% w/v.  Samples were stored at 4°C until assayed.  Cell debris and insoluble material was removed by centrifugation at 1500 G for 10 minutes prior to assay.

## VII.   Assay Protocol

50 ul of patient urine, assay controls, and known assay standards were added to previously prepared $S_{27}$ and control mouse IgG coated microtiter plates.  Urines were incubated for one hour at 25°C.  Nonbound antigen as well as other carbohydrate containing materials were removed by aspiration and the plates were washed three times with PBST.  50 ul of bWGA was added and incubated for one hour at 25°C.  Nonbound lectin was aspirated away and the plates washed another three times with PBST.  50 ul of Avid-HRP was added for one hour followed by aspiration and washing.  50 ul of the enzyme substrate, ortho-phenylenediamine was added and color development was permitted for one hour at 25°C whereupon enzymatic action was halted with the addition of 4.5 M sulfuric acid.  Enzymatic activity was quantitated by measuring color development at 490 nm. Nonspecific binding to control mouse IgG was typically 0.1 to 0.3 optical density units and was subtracted from assay values. A linear standard curve was generated and arbitrary units of reactivity (A.U.) were assigned. One optical density unit at 490 nm was designated as one arbitrary unit. Patient values were determined from this standard curve.

Within assay precision and between assay precision was 12% or less. Normal urine specimens were assayed and had a mean of 0.3 A.U. Patients with kidney disease demonstrated elevated levels of kidney antigen ranging from 0.1 to greater than 7.0 A.U.

Example 2

Modified Assay Protocol

To $S_{27}$ and control mouse IgG coated microtiter plates, prepared pursuant to the methods of Example 1, 50 ul of patient urine, assay controls, and known assay standards were added. Following incubation, nonbound antigen and other carbohydrate containing materials were removed by washing with PBST. 50 ul of wheat germ agglutinin conjugated to horseradish peroxidase (WGA-HRP) (Vector Laboratories Inc.) was added for one hour at 25°C. Nonbound WGA-HRP was removed by washing and color development and quantitation occurs as previously noted in Example 1. Optimal concentrations of WGA-HRP was determined previously as described in Example 1. Using lot number 10912 a concentration of 167 ug/ml was found to be acceptable.

Example 3 - $S_{23}/S_{27}$ Double Antibody Assay Used For Comparison

I.  Monoclonal Antibody Purification

$S_{23}$ was purified from ascitic protein as previously described in Example 1.

II.  Antibody Conjugation

$S_{23}$ was conjugated with horseradish peroxidase (HRP) according to the method of Wilson and Nakane (In Immunofluorescence and Related Staining Techniques, ed W. Knapp et al. p215, 1978).  Free HRP was removed by ammonium sulfate fractionation and further purification of the $S_{23}$-HRP conjugate was accomplished by gel permeation chromatography.  Optimal signal to noise ratios were determined by titering the $S_{23}$-HRP conjugate versus nonspecific mouse IgG, gamma 1 isotype, and $S_{27}$ coated microtiter plates in the assay described below.  Optimal concentration of the $S_{23}$-HRP was found to be 385 ng/ml.

III.  Solid Phase Configuration

$S_{27}$ was adsorbed onto the surfaces of microtiter plates and all conditions were identical to those described in Example 1 with the exception of the washing solution which was a phosphate buffered saline solution containing 0.05% Tween 20 and 0.15 M sodium chloride (PBSS).

IV.  Standard Urine Preparation

Standards were prepared as previously described in Example 1.

CRL-12

## V.  Assay Protocol

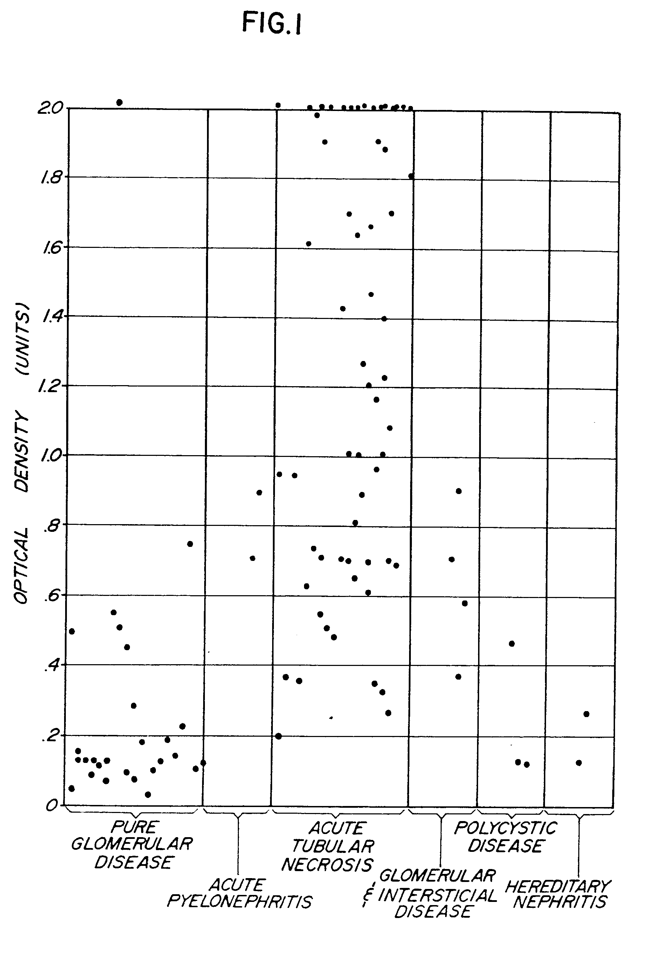

50 ul of patient urine, assay controls, and known assay standards were added to previously prepared $S_{27}$ coated microtiter plates.  Urines were incubated for one hour at 25°C.  Nonbound antigen was aspirated out and the plates were washed three times with PBSS.  $S_{23}$-HRP was added and incubated for one hour at 25°C.  Nonbound conjugated antibody was aspirated out and the plates were washed three times with PBSS prior to the addition of the enzyme substrate ortho-phenylenediamine.  Color development and quantitation was performed as in Example 1 and results shown in Figure 1 for a variety of patient samples.

Within assay precision was 5.2 - 7.2% and between assay precision was 6 - 12%.  Normal urine specimens were assayed and had a mean of 0.1 A.U.  Patients with acute tubular necrosis, or those with recent kidney transplants, demonstrated elevated levels of antigen ranging from 0.5 to more than 10 A.U.

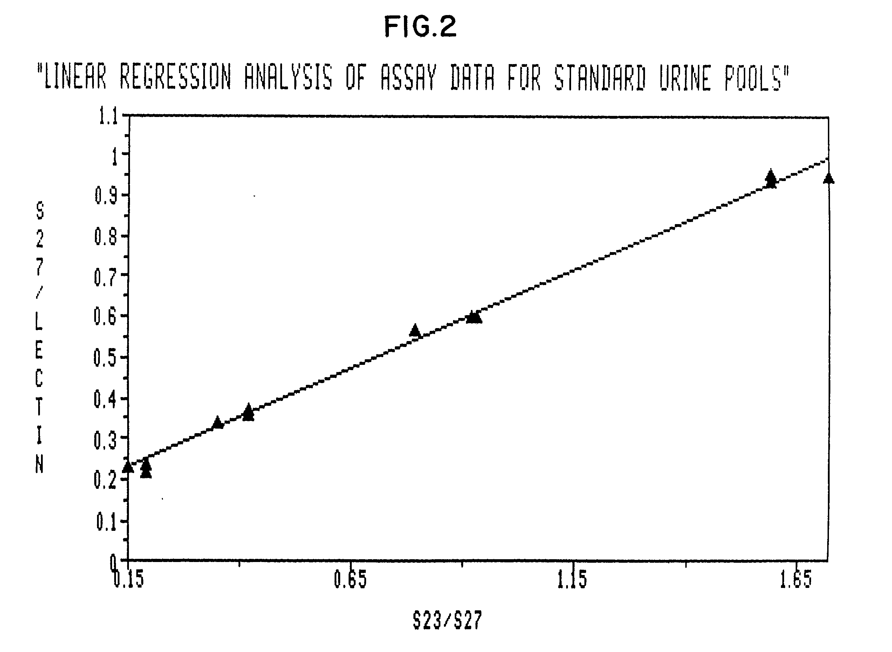

Assay standards prepared from two different standard urine preparations were evaluated in the $S_{27}$/Lectin assay described in Example 1 and in the $S_{23}/S_{27}$ assay described in Example 3.  Data was evaluated by linear regression analysis and a correlation coefficient of 0.99 was obtained for the two assays as shown in Figure 2.

## Example 4 - Kidney Transplantation Patient Study

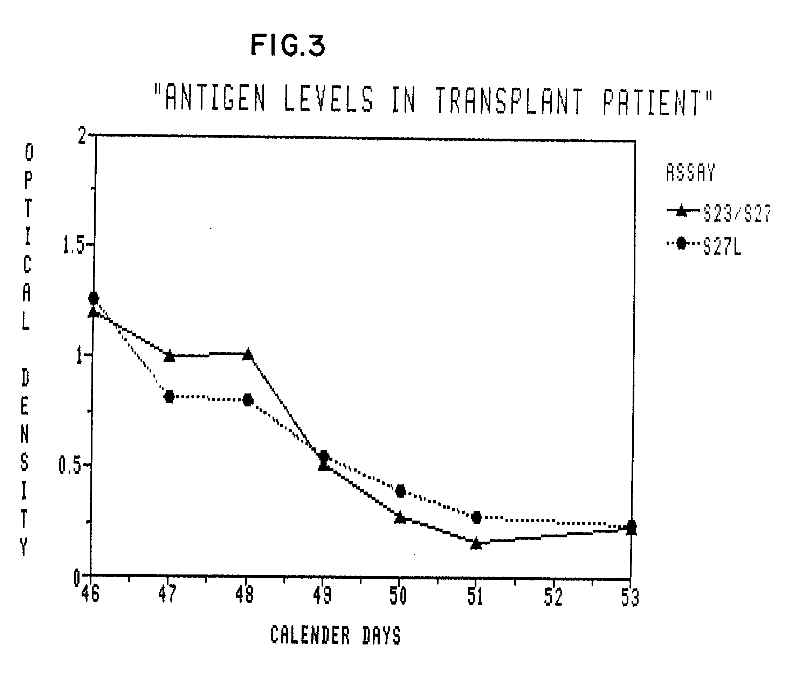

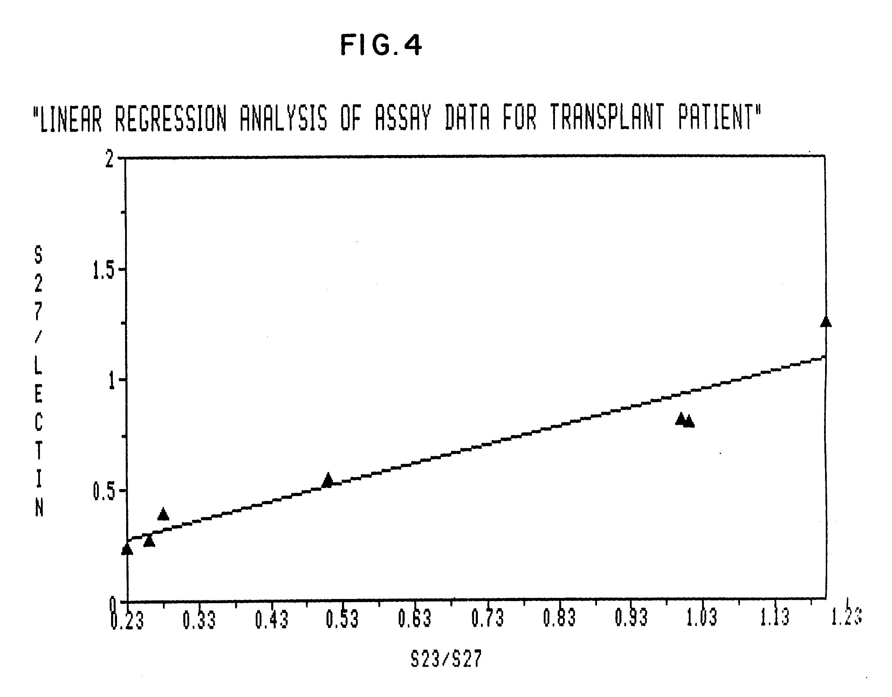

Prospective studies were performed on patients following kidney transplantation to demonstrate the specificity and validity of the monoclonal $S_{27}$ antibody-lectin sandwich assay, described in Example 1, as compared to the dual epitope, monoclonal antibody $S_{23}/S_{27}$ sandwich assay, described in Example 3.  As seen in Figure 3, the levels of antigen measured in urine from a kidney transplant patient using the two assays fluctuates daily in a concordant manner. The clinical significance of this fluctuation is still undergoing evaluation. Linear regression analysis of antigen levels as measured by the two assays, Figure 4, illustrates a good correlation between the two assays with a correlation coefficient of 0.90 for this patient indicating the two assays are measuring the same antigen. Similar results have been observed with other kidney transplant patients as well as patients with acute tubular necrosis.

CLAIMS

1. A method for determining the presence in a sample of a ligand comprising the steps of:

providing a first ligand binding partner specific for said ligand and immobilized onto a solid phase;

contacting said sample with said first immobilized ligand binding partner under conditions permitting an immunological reaction therebetween;

removing substantially all unbound sample components;

contacting said solid phase with a nonspecific agent capable of binding with a moiety associated with said ligand, said nonspecific agent having a detectable label associated therewith;

removing substantially all unbound nonspecific agent; and

detecting the label associated with said solid phase or with said removed unbound nonspecific agent whereby the presence of said ligand in said sample may be determined.

2. The method of claim 1 wherein said moiety is at least one sugar residue and said nonspecific agent is a lectin.

3. The method of claim 2 wherein said lectin is selected from the group consisting of lentil lectin, peanut lectin, conconavalin A, and wheat germ lectin, and said ligand is selected from the group consisting of glycoproteins, glycopeptides, carbohydrates, and glycolipids.

4. The method of any one of claims 1 to 3 wherein said ligand binding partner is selected from the group consisting of antibodies, antibody fragments, analyte receptors, cofactors, and complement components.

5. The method of any one of claims 1 to 4 wherein said solid phase is selected from the group consisting of microtiter well surfaces, plastic surfaces, paddles, microparticles, and macroparticles.

6. The method of any one of claims 1 to 5 wherein said label is selected from the group consisting of phosphorescent molecules, fluorescent molecules, chemiluminescent molecules, light scattering particles, light absorbing dyes, isotopes, and enzymes.

7. The method of any one of claims 1 to 6 wherein said detecting step further comprises comparing said detected level of label with a standard whereby the amount of said ligand present in said sample may be determined.

8. A method for detecting the presence of a sugar, residue containing ligand in an aqueous sample comprising:
immunologically reacting said ligand with a binding partner specific for a determinant associated with said ligand;
reacting a labeled lectin with said ligand whereby said lectin binds to a sugar residue in said ligand; and
detecting the label associated with said ligand which has reacted with said lectin and with said binding partner.

9. The method of claim 8 further comprising the step of removing unreacted aqueous sample prior to said lectin reacting step, and the step of removing unreacted lectin prior to said detecting step.

FIG.1

0166623

# FIG.2

## "LINEAR REGRESSION ANALYSIS OF ASSAY DATA FOR STANDARD URINE POOLS"

# FIG.3

## "ANTIGEN LEVELS IN TRANSPLANT PATIENT"

# FIG.4

"LINEAR REGRESSION ANALYSIS OF ASSAY DATA FOR TRANSPLANT PATIENT"